# EUROPEAN PATENT SPECIFICATION

(11) **EP 2 189 105 B1**
(45) Date of publication and mention of the grant of the patent: **27.06.2012**
(21) Application number: 09176930.7
(22) Date of filing: 24.11.2009
(51) Int. Cl.: A61M 25/00, A61B 1/00, A61B 1/005

(54) **Endoscope**
Endoskop
Endoscope

(30) Priority: 25.11.2008 JP 2008299450
(43) Date of publication of application: 26.05.2010
(73) Proprietor: Fujifilm Corporation, Minato-ku Tokyo 106-8620 (JP)
(72) Inventor: ANDO, Tadashi, Saitama (JP)
(74) Representative: Klunker, Hans-Friedrich

(56) References cited:
- EP-A- 0 861 674
- WO-A-2008/139847
- DE-A1-102007 001 580
- US-A1- 2005 054 898
- US-A1- 2007 162 101
- US-A1- 2008 208 001

## Description

### 1. Technical Field

The invention relates to an endoscope in which an outer peripheral surface of a bendable portion provided on a distal end side of an endoscope insertion part is covered with an elastic tube having flexibility.

### 2. Description of the Related Art

An endoscope has an endoscope insertion part which is inserted into a body cavity or the like, and a operation part which is provided to be continued from a proximal end side of the endoscope insertion part. The endoscope insertion part is configured so that a distal end hard portion made of a hard member, a bendable portion which can be operated to be bent, and an introduction portion are continuously provided in this order from the distal end side. The distal end hard portion has a distal end portion body including an observation optical system for observing an inside of a body cavity, and a distal end sleeve which is fitted and fixed to the distal end portion body. A base end of the distal end sleeve is connected with the bendable portion. The bendable portion has a joint ring structure in which a number of joint rings are connected continuously and pivotally. The outer peripheries of the distal end hard portion and the bendable portion are covered with an elastic tube having flexibility, thereby airtightly holding the distal end hard portion and the bendable portion.

The above described elastic tube is fixed in two places of the distal end and base end thereof, and is not necessarily fixed in a range from the distal end to the base end. Therefore, for example, when dirt adhered to the endoscope insertion part is wiped and cleaned after endoscopic examination, the elastic tube may be pulled to the distal end due to the load (squeeze, etc.) applied during the cleaning, and deviation and loosening may occur in the elastic tube. Once the elastic tube is loosened, the tube does not naturally return to its original state even if the load is released. If the loosening exists, the elastic tube which has been pulled in the longitudinal direction may be folded back, and may be covered on the distal end. As a result, a diameter of the distal end becomes locally large, and it may become difficult to smoothly move the endoscope insertion part within the body cavity.

JP Sho.58-49132 A describes a technique regarding an endoscope having a first bendable portion which is bent by a bending operation performed by an operation part, and a second bendable portion which is bent only by an external force. In this endoscope, loosening of the elastic tube is prevented by fixing a portion of the elastic tube (angle rubber) at a boundary portion between the first bendable portion and the second bendable portion.

However, there is a disadvantage that a step portion (height difference) is caused as the elastic tube is fixed at the boundary portion between the first bendable portion and the second bendable portion, and the diameter of the endoscope insertion part increases due to this step portion. Additionally, it is necessary to provide a recess at the boundary portion so that the elastic tube does not deviate. Due to this recess, there is a disadvantage that the internal diameter of the bendable portion is forced to be small, and a section area used to house conduits or the like is reduced.

### SUMMARY OF THE INVENTION

The invention has been made in view of the above circumstances, and provides an endoscope which can prevent deviation and loosening of an elastic tube covering an outer periphery of a bendable portion of an endoscope insertion part.

According to an aspect of the invention, an endoscope includes an endoscope insertion part, a bendable portion and an elastic tube. The bendable portion is configured to be operated to be bent within one plane which is parallel to an axial direction of the endoscope insertion part. The bendable portion is disposed on a distal end side of the endoscope insertion part. The elastic tube has flexibility and covers an outer peripheral surface of the bendable portion. A rigidity of the elastic tube in directions orthogonal to bending operation directions is higher than that of the elastic tube in the bending operation directions.

With the above endoscope, it is possible to prevent deviation and loosening of the elastic tube covering the outer periphery of the bendable portion of the endoscope insertion part.

### BRIEF DESCRIPTION OF THE DRAWINGS

Fig. 1 is an appearance perspective view of an endoscope according to an embodiment of the invention.
Fig. 2 is a section view showing a bendable portion of Fig. 1 taken along a line A-A in a state where a housed object is omitted.
Fig. 3 is a conceptual diagram showing a bendable tube structure.
Fig. 4A is a perspective view of an angle rubber alone.
Fig. 4B is a section view taken along a line B-B.
Fig. 5 is an explanatory view conceptually showing a state where the bendable portion is bent.
Fig. 6A is a section view of the angle rubber equivalent to the section B-B of Fig. 4A.
Fig. 6B is a section view showing the bendable portion when the angle rubber covers the insertion part of the endoscope, in a state where a housed object is omitted.
Fig. 7A is a section view of the angle rubber equivalent to the section B-B of Fig. 4A.
Fig. 7B is a section view showing the bendable portion when the angle rubber covers the insertion part of the endoscope, in a state where a housed object is omitted.
Fig. 8A is a section view of the angle rubber equivalent to the section B-B of Fig. 4A
Fig. 8B is a section view showing the bendable portion when the angle rubber covers the insertion part of the endoscope, in a state where a housed object is omitted.
Fig. 9A is a section view of the angle rubber equivalent to the section B-B of Fig. 4A.
Fig. 9B is a section view showing the bendable portion when the angle rubber covers the insertion part of the endoscope, in a state where a housed object is omitted.
Fig. 10 is a section view showing the bendable portion when the angle rubber in which a hard portion is made thin covers the insertion part of the endoscope, in a state where a housed object is omitted.

### DETAILED DESCRIPTION OF EMBODIMENTS OF THE INVENTION

Hereinafter, an endoscope will be described in detail with reference to the drawings.

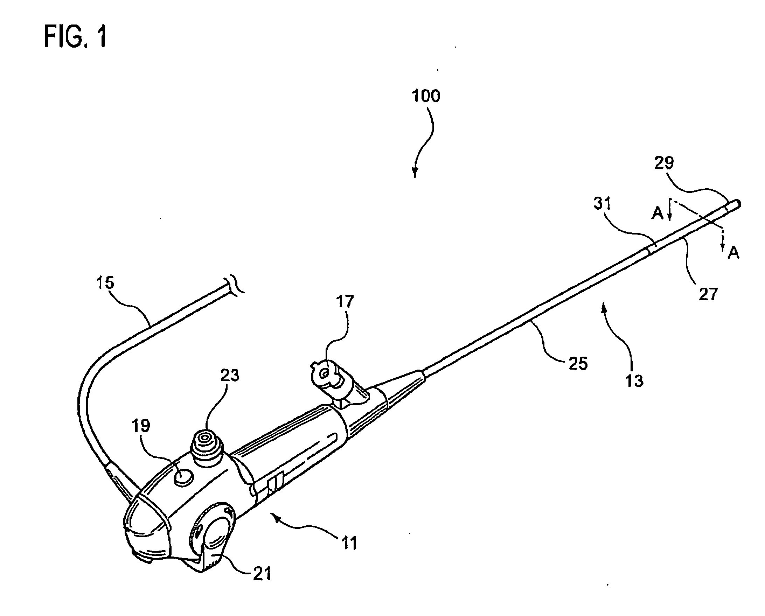

Fig. 1 is an appearance perspective view of the endoscope for explaining the embodiment of the invention.

The endoscope 100 of this configuration mainly has a operation part 11, and an endoscope insertion part 13 which extends to the operation part 11. The endoscope insertion part 13 includes channel holes, various types of conduits, etc. therein. A light guide (LG) flexible part 15 is connected to the operation part 11. The light guide flexible part 15 is connected to a processor provided with a light source and a signal processor (which are not shown).

The operation part 11 is provided with a forceps opening 17 into which treatment tools, such as forceps, are inserted. The operation part 11 is also provided with a switch 19 which is used to fetch an image and to switch among functions, an upper and lower angle lever 21 and a suction button 23.

The endoscope insertion part 13 includes a soft portion 25, a bendable portion 27, and a distal end portion 29 in order from a proximal end side which is continued to the operation part 11. The bendable portion 27 located on the distal end side of the endoscope insertion part 13 is remotely operated to be bent, by operating the upper and lower angle lever 21 provided in the operation part 11, to thereby direct the distal end portion 29 toward a desired direction. That is, the bendable portion 27 is operated to be bent in a plane which is parallel to the axial direction of the endoscope insertion part 13. Also, the outer peripheral surfaces of the bendable portion 27 and the distal end portion 29 are covered with an elastic tube (hereinafter referred to as an "angle rubber") 31 having flexibility.

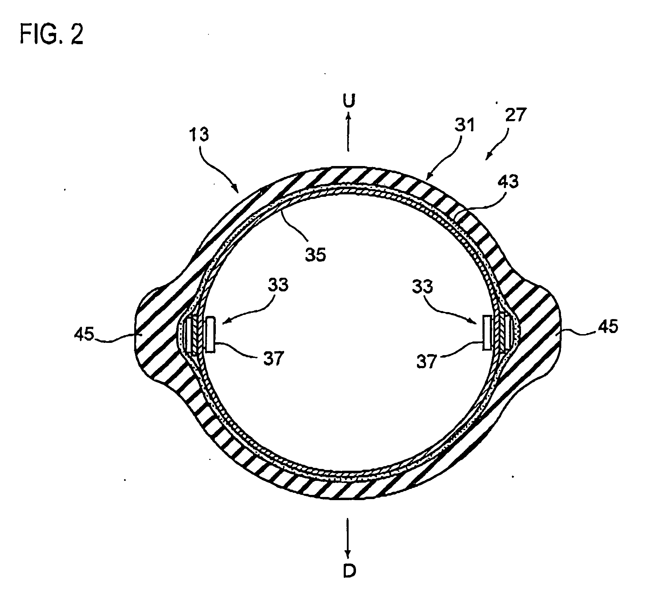

Fig. 2 is a section view showing a section of the bendable portion of Fig. 1 taken along a line A-A, in a state where a housed object is omitted.

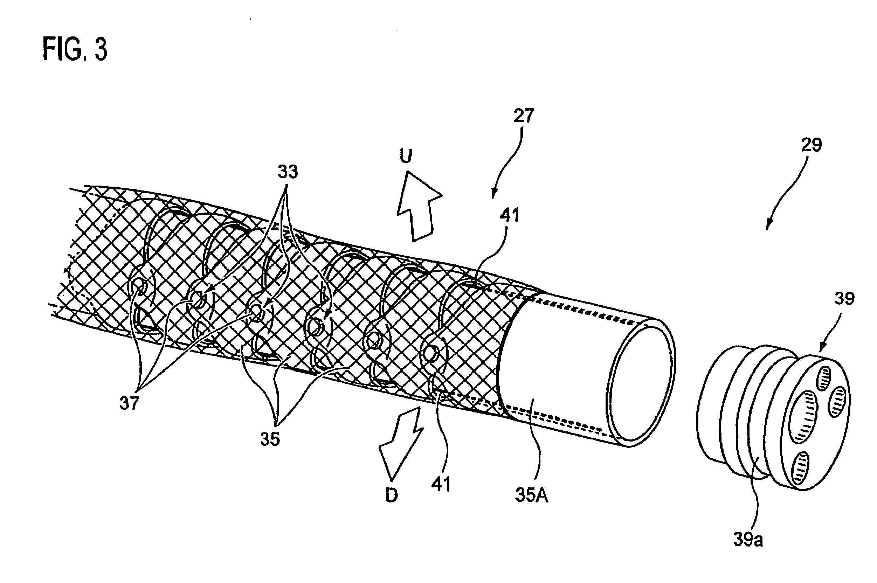

The bendable portion 27 has a bendable tube structure in which a plurality of ring-shaped joint rings 35, each formed with connecting portions 33 on sides orthogonal to the bending operation directions U and D, are juxtaposed along the axial direction of the endoscope insertion part 13, and adjacent joint rings 35 are rotatably connected to each other by the connecting portions 33. Fig. 3 shows this bendable tube structure.

The bendable portion 27 is configured so that the plurality of joint rings 35 formed in a ring shape are continuously pivoted in the axial direction. In adjacent joint rings 35, their connecting portions 33 are overlapped with each other, and then, caulking pins 37 are inserted into through holes provided in the connecting portions 33, and the outsides of the caulking pins 37 are caulked. Thereby, the respective joint rings 35 are rotatably connected together.

Among the joint rings 35, which are continuously pivoted, a joint ring 35A on the most distal end side is formed to be longer in the axial direction. A distal end hard portion 39 made of ceramics or the like is joined to the distal end side, in the axial direction, of the joint ring 35A. Also, a tubular net 43 made of braided metal wire or the like is mounted on the outsides of the joint rings 35 via lubricant. As shown in Fig. 2, the outer periphery of the net 43 is covered with the angle rubber 31. The tip of the net 43 is fixed to the outer periphery of the joint ring 35A on the distal end side. A cylindrical sleeve may be interposed between the joint ring 35A on the distal end side and the distal end hard portion 39.

Inside the joint rings 35, a pair of upper and lower control wires 41 is disposed along the axial direction of the inner peripheral surface thereof. The tips of the control wires 41 and 41 are fixed to the joint ring 35A on the most distal end side. The base ends of the control wires 41 and 41 are connected to pulleys (not shown) rotated by the upper and lower angle lever 21 (see Fig. 1) of the operation part 11. With this configuration, when the upper and lower angle lever 21 is operated to rotate the pulleys, any one of the control wires 41 and 41 is pulled, and the bendable portion 27 is bent in a desired direction.

The angle rubber 31 is a covering member which covers the outer peripheries of the distal end portion 29 and the bendable portion 27 (see Fig. 1), and is made of an elastic material having flexibility. The angle rubber 31 can be formed from, for example, rubber having fluorine as its material. The tip of the angle rubber 31 is fixed, for example, by causing the tip to abut against the distal end hard portion 39 beyond the joint ring 35A on the distal end side, firmly winding a thread around the outer periphery of the angle rubber 31 in a position of a thread-winding portion 39a formed in the distal end hard portion 39, and applying an adhesive or the like thereto. The base end of the angle rubber 31 is fixed to the base end of the bendable portion 27. By fixed the both ends of the angle rubber 31 in this manner, the inside of the angle rubber 31 is airtightly kept.

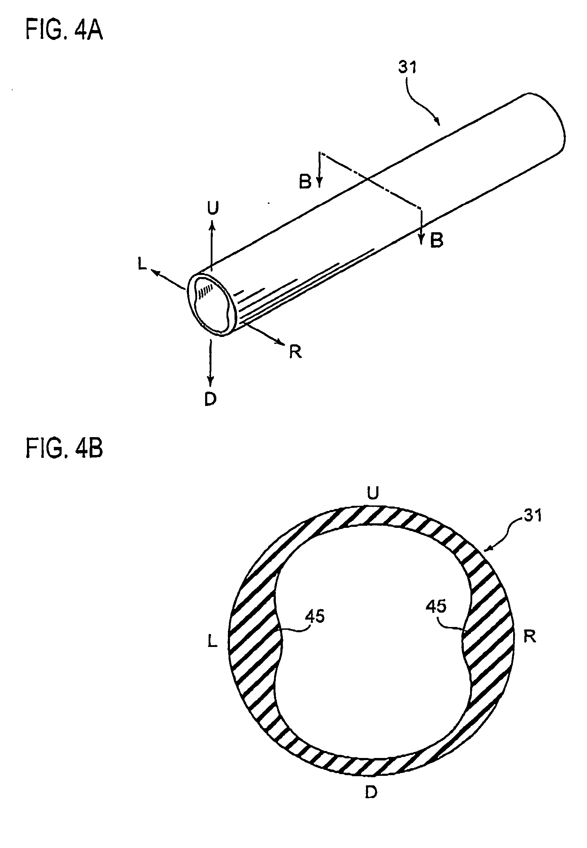

Fig. 4A is a perspective view of the angle rubber alone, and Fig. 4B is a section view taken along a line B-B.

The angle rubber 31 is cylindrical as a whole and is formed with, in the inner peripheral surface thereof, thick-walled portions 45 in directions L and R orthogonal to the bending operation directions U and D. By forming the wall thicknesses of the angle rubber 31 in circumferential positions of the angle rubber 31 in the directions L and R orthogonal to the bending operation directions U and D to be larger than those of the angle rubber 31 in circumferential positions of the angle rubber 31 in the bending operation directions U and D in this manner, the rigidity of the angle rubber 31 in the directions L and R orthogonal to the bending operation directions is higher than that of the angle rubber 31 in the bending operation directions U and D.

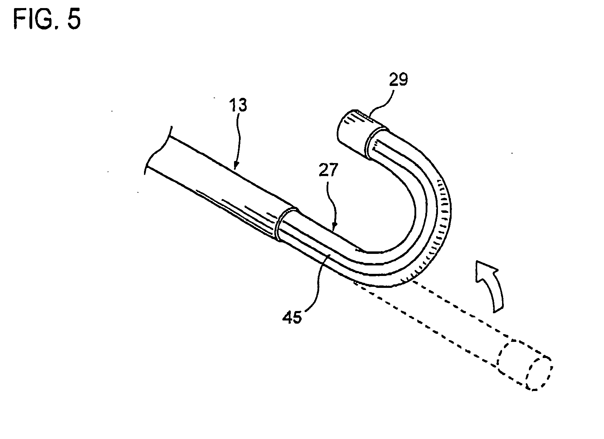

Here, the angle rubber 31 shown in Fig. 4 is aligned with and put on the endoscope insertion part 13 so that the thick-walled portions 45 are located in the circumferential positions of the angle rubber 31 where the thick-walled portions 45 face the connecting portions 33. Then, as shown in Fig. 2, the angle rubber 31 covers the endoscope insertion part 13 in a state where the thick-walled portions 45 protrude toward the outer peripheral side. The existence of the thick-walled portions 45 provides the following effects. That is, the angle rubber 31 is prevented from being pulled to the distal end portion due to a load applied at the time of cleaning when dirt adhered to the endoscope insertion part 13 after endoscopic examination is wiped and cleaned, and deviation and loosening of the angle rubber 31 hardly occur. Moreover, when the bendable portion 27 shown in Fig. 5 is bent, the thick-walled portions 45 are located on the neutral axis of bending displacement. Therefore, bending rigidity does not increase due to the thick-walled portions 45 of the angle rubber 31, to adversely affect the bending operation. Also, the thick-walled portions 45 of the angle rubber 31 are continuously formed along the insertion direction of the endoscope insertion part 13, and are projections which are smooth even in a peripheral direction. Therefore, a stepped portion accompanied by the insertion operation does not influence the thick-walled portions 45.

As described above, deviation and loosening of the angle rubber 31 are prevented by making the rigidity of the angle rubber 31 in the circumferential positions of the angle rubber 31 in the directions L and R orthogonal to the bending operation directions U and D to be higher than that of the angle rubber 31 in the circumferential positions of the angle rubber 31 in the bending operation directions U and D. With this configuration, it is possible to smoothly move the endoscope insertion part 13 within a body cavity, and smooth endoscopy can be always stably carried out.

Next, another example of the above angle rubber 31 will be described.

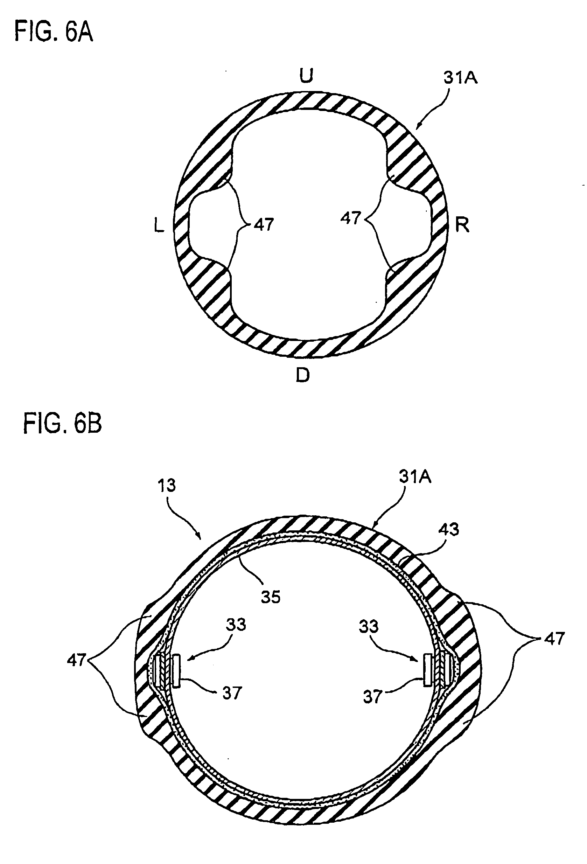

Fig. 6A is a section view of the angle rubber equivalent to the section B-B of Fig. 4A, and Fig. 6B is a section view showing the bendable portion when the angle rubber covers the insertion part of the endoscope, in a state where a housed object is omitted. In the angle rubber 31A of this example, the wall thicknesses of the angle rubber 31A in circumferential positions of the angle rubber 31 A in the directions L and R orthogonal to the bending operation directions U and D is larger than those in circumferential positions of the angle rubber 31A in the bending operation directions U and D. Also, the wall thicknesses of the angle rubber 31A in circumferential positions of the angle rubber 31A which face the connecting portions 33 are smaller than those on both sides of the circumferential positions of the angle rubber 31A which face the connecting portions 33. That is, thick-walled portions 47 are formed on the both sides of the circumferential positions of the angle rubber 31A which face the connecting portions 33 in the orthogonal directions L and R so that an average wall thicknesses in the circumferential positions of the angle rubber 31A in the orthogonal directions L and R are larger than those in the circumferential positions of the angle rubber 31 A in the bending operation directions U and D. Also, the circumferential positions of the angle rubber 31A which face the connecting portions 33 are thinner than the thick-walled portions 47. When the angle rubber 31A of this configuration is aligned with and put on the endoscope insertion part 13 so that the thick-walled portions 47 are located in circumferential positions which sandwich the connecting portions 33, as shown in Fig. 6B, the outermost diameter of the endoscope insertion part 13 in the circumferential positions corresponding to the connecting portions 33 can be made smaller than that of the aforementioned example shown in Fig. 2. Also, the "wall thickness in the circumferential positions in the above orthogonal directions L and R" may mean an average thickness (including the thick-walled portions 47) of the angle rubber 31 A in the orthogonal directions L and R within a predetermined circumferential length.

In the above configuration, with regard to protruding portions of the caulking pins 37 in the connecting portions 33, the protruding portions can be housed by forming the thick-walled portions 47 of the angle rubber 31A to be partially thin. This suppresses an increase in the external diameter of the endoscope insertion part 13 while preventing the loosening of the angle rubber 31A.

Next, further another example of the angle rubber 31 will be described.

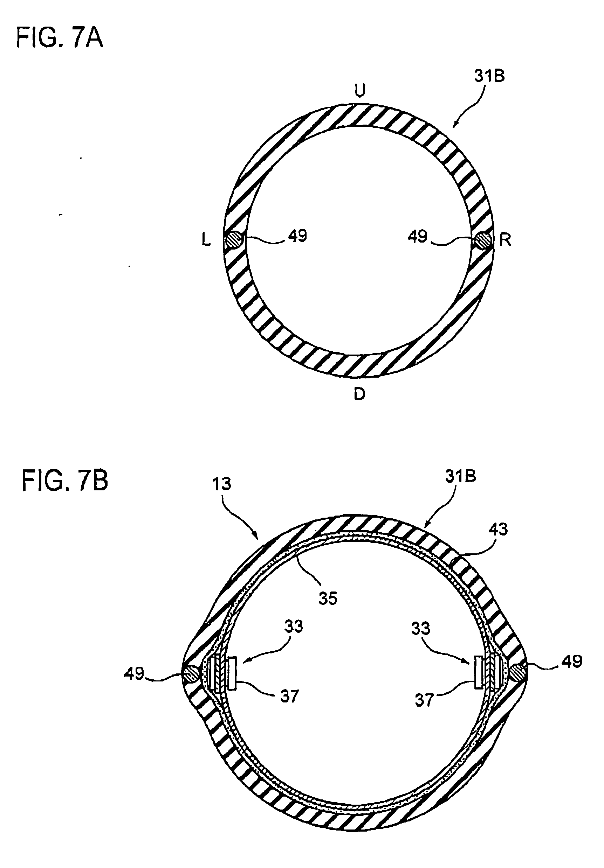

Fig. 7A is a section view of the angle rubber equivalent to the section B-B of Fig. 4A, and Fig. 7B is a section view showing the bendable portion when the angle rubber covers the insertion part of the endoscope, in a state where a housed object is omitted. In the angle rubber 31B in this case, wires 49 are buried along the axial direction of the endoscope insertion part 13 in the angle rubber 31B in the circumferential positions of the angle rubber 31B in the directions L and R orthogonal to the bending operation directions U and D. Although not shown, both ends of the wires 49 are fixed to the angle rubber 31B. By arranging the wires 49 in the angle rubber 31B in the circumferential positions corresponding to the connecting portions 33, the rigidity of the angle rubber 31B in circumferential positions of the angle rubber in the directions orthogonal to the bending operation directions is higher than that in circumferential positions of the angle rubber 31B in the bending operation directions of the angle rubber 31B. As a result, deviation and loosening of the angle rubber 31B are prevented from occurring. Moreover, since there is no thick-walled portion in the bending operation direction of the angle rubber 31B, and the wires 49 exist on the neutral axis of the bending operation, the bending operation is not adversely affected. Additionally, the durability of the angle rubber 31B itself can be improved by integrally forming the hard wires 49.

In the illustrated example, the wires 49 are arranged in the circumferential positions of the angle rubber 31 B in the directions L and R orthogonal to the bending operation directions U and D, respectively. However, the invention is not limited thereto. A plurality of wires (the number of wires being arbitral) may be provided. For example, by burying a plurality of wires in regions which face the caulking pins 37, the angle rubber 31b can be reinforced so that a portion where an external force becomes apt to be concentrated is covered by the protrusion of the caulking pins 37, and durability can be further improved. If the section shape of the wires 49 is formed in a rectangular shape other than a circular shape, a region where rigidity becomes high can be expanded in the peripheral direction of the angle rubber 31B, and the durability of the angle rubber 31B is further improved.

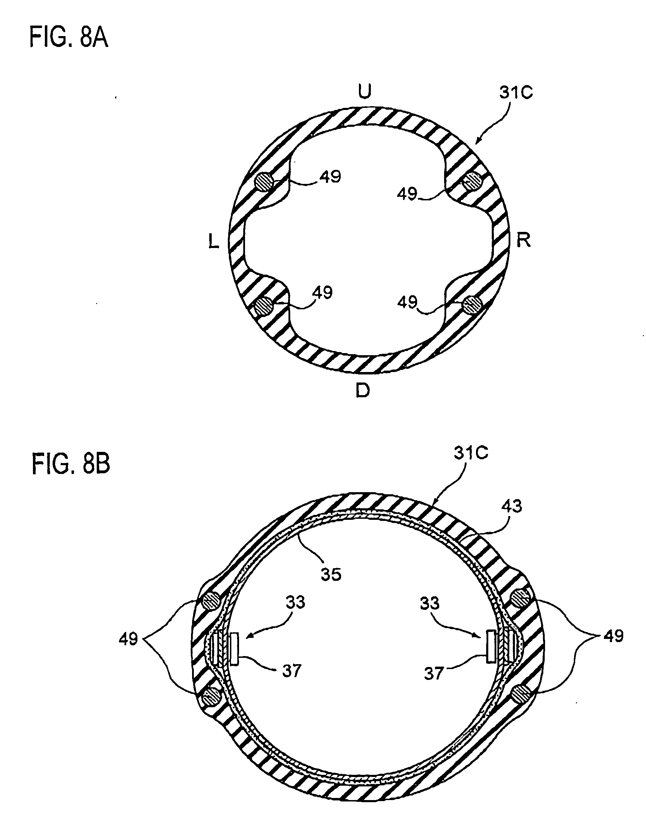

Still further another configuration example in which wires are buried within the thick-walled portions of the angle rubber is shown in Figs. 8A and 8B.

Fig. 8A is a section view of the angle rubber equivalent to the section B-B of Fig. 4A. Fig. 8B is a section view showing the bendable portion when the angle rubber covers the insertion part of the endoscope in a state where a housed object is omitted. The angle rubber 31C of this example is configured so that wires 49 are respectively buried in thick-walled portions 47 of the angle rubber 31A shown in Figs. 6A and 6B.

In this case, a plurality of wires 49 are respectively arranged in circumferential positions of the angle rubber 31 C in the directions L and R orthogonal to the bending operation directions U and D of the angle rubber 31 C. Therefore, the resistance of the angle rubber 31C against twist in the axial direction becomes high, and loosening can be made less likely to occur.

Next, still further another example of the angle rubber will be described.

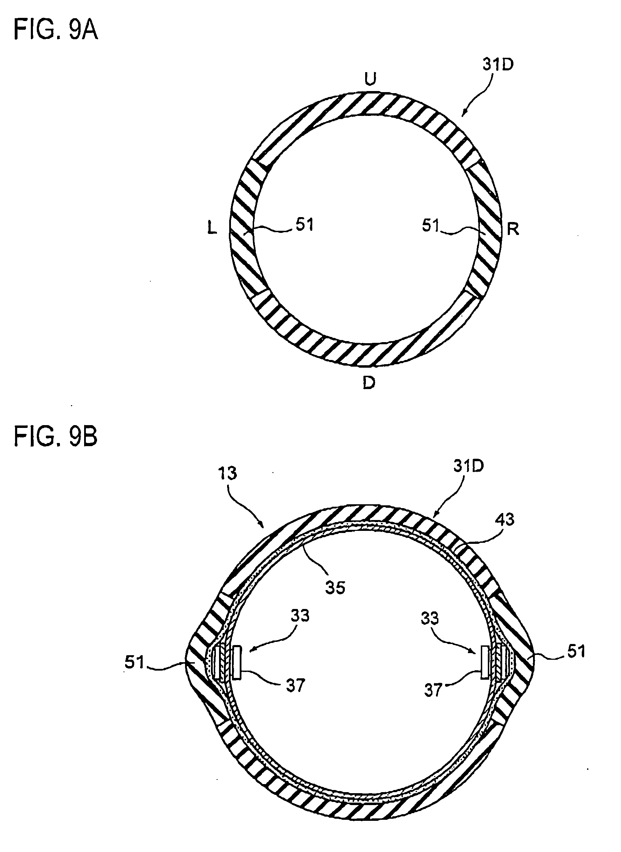

Fig. 9A is a section view of the angle rubber equivalent to the section B-B of Fig, 4A. Fig. 9B is a section view showing the bendable portion when the angle rubber covers the insertion part of the endoscope, in a state where a housed object is omitted. The angle rubber 31D of this example is configured so that portions in the circumferential positions of the angle rubber 31D in the directions L and R orthogonal to the bending operation directions U and D are formed from an elastic material having a larger elastic constant than that of portions of the angle rubber 31D in the other circumferential positions. That is, the angle rubber 31D is formed from materials having elastic constants which are different from each other, in the circumferential positions of the angle rubber 31D in the bending operation directions U and D and in the circumferential positions of the angle rubber 31D in the orthogonal directions L and R. The angle rubber 31D includes hard portions 51 in the circumferential positions of the angle rubber 31D in the orthogonal directions L and R. The hard portions 51 may be formed from a harder rubber material or the like than in the other circumferential positions of the angle rubber 31D. The angle rubber 31D having such hard portions 51 can be efficiently molded by, for example, processing methods, such as multi-color extrusion molding which simultaneously extrudes and molds elastic materials having mutually different elastic constants, or a combined extrusion molding of manufacturing hard portions 51 in advance and extrudes and molding the hard portions 51 from a softer elastic material.

According to the angle rubber 31D, the rigidity can be changed while the radial thickness is kept constant. The angle rubber 31D can be processed at low cost while productivity is increased by suppressing the amount of raw material to a required minimum. In the case where the hard portions 51 which are molded in advance are insert-molded as in the above combined extrusion molding, local processing of the hard portions 51, such as forming recesses which house the protruding caulking pins 37 in the inner peripheral surface, can be simply performed.

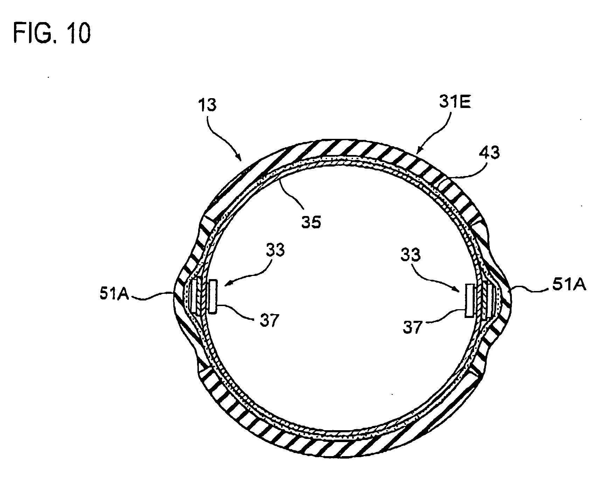

Moreover, by making hard portions 51A thinner than the other portions like an angle rubber 31E shown in Fig. 10, protruding of the caulking pins 37 in the connecting portions 33 can be prevented from becoming higher than the other circumferential positions.

As described above, the following matters are described in this specification. (1) An endoscope includes an endoscope insertion part, a bendable portion and an elastic tube. The bendable portion is configured to be operated to be bent within one plane which is parallel to an axial direction of the endoscope insertion part. The bendable portion is disposed on a distal end side of the endoscope insertion part. The elastic tube has flexibility and covers an outer peripheral surface of the bendable portion. A rigidity of the elastic tube in directions orthogonal to bending operation directions is higher than that of the elastic tube in the bending operation directions.

With this endoscope, occurrence of deviation and loosening of the elastic tube can be prevented, and the bending operation is not adversely affected while the bending operation can be operated with a small load. (2) In the endoscope of (1), wall thicknesses of the elastic tube in circumferential positions of the elastic tube in the directions orthogonal to the bending operation directions may be larger than those of the elastic tube in circumferential positions of the elastic tube in the bending operation directions.

With this endoscope, the rigidity of the elastic tube can be easily adjusted with a high degree of freedom by varying wall thicknesses of the elastic tube. Also, the durability of the elastic tube is improved by the existence of a portion whose wall thickness is large. (3) In the endoscope of any one of (1) to (2), wires may be buried along the axial direction in the elastic tube in circumferential positions of the elastic tube in the directions orthogonal to the bending operation directions.

With this endoscope, the rigidity of the elastic tube is increased by burying the wires without great increase in diameter of the endoscope insertion part. (4) In the endoscope of (2), the bendable portion may have a bendable tube structure. In the bendable structure, a plurality of ring-shaped joint rings each formed with connecting portions in circumferential positions thereof in the directions orthogonal to the bending operation directions are juxtaposed along the axial direction, and adjacent joint rings are rotatably connected to each other by the connecting portions. The wall thicknesses of the elastic tube in the circumferential positions of the elastic tube which face the connecting portions may be smaller than those of the elastic tube on both sides of the circumferential positions of the elastic tube which face the connecting portions.

With this endoscope, the protruding height of the elastic tube to the outside can be suppressed to be low by making the elastic tube be thin in the connecting portions which protrude in the axial direction. (5) In the endoscope of (4), wires may be buried along the axial direction in thick-walled portions of the elastic tube on the both sides of the circumferential positions of the elastic tube which face the connecting portions.

With this endoscope, the wires are arranged on the both sides sandwiching the connecting portions by burying the wires in the thick-walled portions, respectively. Thus, the strength of the endoscope insertion part against twist in the axial direction increases, and the durability improves. Additionally, the wires can be arranged with space efficiency being enhanced. (6) In the endoscope of (1), the circumferential positions of the elastic tube in the directions orthogonal to the bending operation directions may be formed from an elastic material having a larger elastic constant than other circumferential positions of the elastic tube.

With this endoscope, the angle rubber can be processed at low cost while productivity is increased by suppressing the amount of raw material to a required minimum.

The above description on the embodiments of the invention has been provided for the purposes of illustration and description. It is not intended to be exhaustive or to limit the invention thereto. Various modifications will be apparent to one skilled in the art.

## Claims

1. An endoscope comprising:
an endoscope insertion part (13); and
a bendable portion (27) configured to be operated to be bent within one plane which is parallel to an axial direction of the endoscope insertion part (13), the bendable portion (27) that is disposed on a distal end side of the endoscope insertion part (13); **characterized by**
an elastic tube (31) that has flexibility and covers an outer peripheral surface of the bendable portion (27), wherein
a rigidity of the elastic tube (31) in directions orthogonal to bending operation directions is higher than that of the elastic tube (31) in the bending operation directions.

2. The endoscope according to claim 1, wherein wall thicknesses of the elastic tube (31) in circumferential positions of the elastic tube (31) in the directions orthogonal to the bending operation directions are larger than those of the elastic tube (31) in circumferential positions of the elastic tube (31) in the bending operation directions.

3. The endoscope according to any one of claims 1 to 2, wherein wires (49) are buried along the axial direction in the elastic tube (31) in circumferential positions of the elastic tube (31) in the directions orthogonal to the bending operation directions.

4. The endoscope according to claim 2, wherein
the bendable portion (27) has a bendable tube structure in which
a plurality of ring-shaped joint rings (35) each formed with connecting portions (33) in circumferential positions thereof in the directions orthogonal to the bending operation directions are juxtaposed along the axial direction, and
adjacent joint rings (35) are rotatably connected to each other by the connecting portions (33), and
the wall thicknesses of the elastic tube (31) in the circumferential positions of the elastic tube (31) which face the connecting portions (33) are smaller than those of the elastic tube (31) on both sides of the circumferential positions of the elastic tube (31) Which face the connecting portions (33).

5. The endoscope according to claim 4, wherein wires (49) are buried along the axial direction in thick-walled portions of the elastic tube (31) on the both sides of the circumferential positions of the elastic tube (31) which face the connecting portions (33).

6. The endoscope according to claim 1, wherein the circumferential positions of the elastic tube (31) the directions orthogonal to the bending operation directions are formed from an elastic material having a larger elastic constant than other circumferential positions of the elastic tube.

## Patentansprüche

1. Endoskop, umfassend:
ein Endoskopeinführteil (13); und
einen biegbaren Abschnitt (27), konfiguriert, um betätigt zu werden zum Biegen innerhalb einer Ebene, die parallel ist zu einer axialen Richtung des Endoskopeinführteils (13), wobei der biegbare Abschnitt (27) sich auf einer distalen Endseite des Endoskopeinführteils (13) befindet,
**gekennzeichnet durch**
einen elastischen Schlauch (31), der Flexibilität besitzt und eine Außenumfangsfläche des biegbaren Abschntits (27) abdeckt, wobei
die Steifigkeit des elastischen Schlauchs (31) in Richtungen orthogonal zu den Biegerichtungen höher ist als die des elastischen Schlauchs (31) in die Biegerichtungen.

2. Endoskop nach Anspruch 1, bei dem die Wandstärke des elastischen Schlauchs (31) an Umfangsstellen des elastischen Schlauchs (31) in die Richtungen orthogonal zu den Biegerichtungen größer ist als jene dies elastischen Schlauchs (31) an Umfangsstellen des elastischen Schlauchs (31) in den Biegerichtungen.

3. Endoskop nach einem der Ansprüche 1 bis 2, bei dem Drähte (49) entlang der axialen Richtung in dem elastischen Schlauch (31) an Umfangsstellen des elastischen Schlauchs (31) in Richtungen orthogonal zu den Biegerichtungen eingebettet sind.

4. Endoskop nach Anspruch 2, bei dem der biegbare Abschnitt (27) eine biegbare Schlauchstruktur aufweist, in der
eine Mehrzahl von ringförmigen Verbindungsringen (35) die jeweils mit Verbindungsabschnitten (33) in deren Umfangsrichtungen in den Richtungen orthogonal zu den Biegerichtungen entlang der axialen Richtung nebeneinander angeordnet sind, und
benachbarte Verbindungsringe (35) drehbar miteinander über die Verbindungsabschnitte (33) verbunden sind, und
die Wandstärken des elastischen Schlauchs (31) an den Umfangsstellen des elastischen Schlauchs (31), die den Verbindungsabschnitten (33) gegenüberliegen, geringer sind als jene des elastischen Schlauchs (31) auf beiden Seiten der Umfangsstellen des elastischen Schlauchs (31), die den Verbindungsabschnitten (33) gegenüberliegen.

5. Endoskop nach Anspruch 4, bei dem entlang der axialen Richtung in dickwandigen Abschnitten des elastischen Schlauchs (31) auf den beiden Seiten der Umfangsstellen des elastischen Schlauchs (31), die den Verbindungsabschnitten (33) gegenüberliegen, Drähte (49) eingebettet sind.

6. Endoskop nach Anspruch 1, bei dem die Umfangsstellen des elastischen Schlauchs (31) in den Richtungen orthogonal zu den Biegerichtungen aus einem elastischen Werkstoff gebildet sind, der eine größere Elastizitätskonstante aufweist als die anderen Umfangsstellen des elastischen Schlauchs.

## Revendications

1. Endoscope comprenant :
une partie d'insertion d'endoscope (13) ; et
une partie courbable (27) configurée pour être utilisée pour être courbée dans un plan qui est parallèle à une direction axiale de la partie d'insertion d'endoscope (13), la partie courbable (27) étant disposée sur un côté d'extrémité distale de la partie d'insertion d'endoscope (13) ; **caractérisé par**
un tube élastique (31) qui a une certaine flexibilité et couvre une surface périphérique extérieure de la partie courbable (27), dans lequel
une rigidité du tube élastique (31) dans des directions orthogonales aux directions de courbure est supérieure à celle du tube élastique (31) dans les directions de courbure.

2. Endoscope selon la revendication 1, dans lequel les épaisseurs de paroi du tube élastique (31) dans des positions circonférentielles du tube élastique (31) dans des directions orthogonales aux directions de courbure sont supérieures à celles du tube élastique (31) dans des positions circonférentielles du tube élastique (31) dans les directions de courbure.

3. Endoscope selon l'une quelconque des revendications 1 à 2, dans lequel des fils métalliques (49) sont enfouis le long de la direction axiale dans le tube élastique (31) dans des positions circonférentielles du tube élastique (31) dans des directions orthogonales aux directions de courbure.

4. Endoscope selon la revendication 2, dans lequel
la partie courbable (27) a une structure de tube courbable dans laquelle
une pluralité de joints circulaires en forme d'anneau (35) chacun formé avec des parties de connexion (33) dans des positions circonférentielles de ceux-ci dans les directions orthogonales aux directions de courbure sont juxtaposés le long de la direction axiale, et
des joints circulaires (35) adjacents sont connectées à rotation les uns aux autres par les parties de connexion (33), et
les épaisseurs de paroi du tube élastique (31) dans les positions circonférentielles du tube élastique (31) qui font face aux parties de connexion (33) sont plus petites que celles du tube élastique (31) sur les deux côtés des positions circonférentielles du tube élastique (31) qui font face aux parties de connexion (33).

5. Endoscope selon la revendication 4, dans lequel des fils métalliques (49) sont enfouis le long de la direction axiale dans des parties de paroi épaisse du tube élastique (31) sur les deux côtés des positions circonférentielles du tube élastique (31) qui font face aux parties de connexion (33).

6. Endoscope selon la revendication 1, dans lequel les positions circonférentielles du tube élastique (31) dans les directions orthogonales aux directions de courbure sont formées à partir d'un matériau élastique ayant une constante d'élasticité plus grande que d'autres positions circonférentielles du tube élastique.
